# EUROPEAN PATENT APPLICATION

(11) **EP 4 245 223 A1**
(43) Date of publication of application: **20.09.2023**
(21) Application number: 21903674.6
(22) Date of filing: 22.11.2021
(51) Int. Cl.: A61B 8/00, A61L 2/10, A61L 2/14

(54) **MEDICAL PROBE STERILIZER**

(30) Priority: 07.12.2020 KR 20200169527
(71) Applicant: IBMsol Co., Ltd., Seoul 02447 (KR)
(72) Inventor: KIM, Hee Kyung, Seoul 01867 (KR)
(74) Representative: Krauns, Christian
(86) International application number: PCT/KR2021/017125
(87) International publication number: WO 2022/124628

(57) **Abstract**

Proposed is a medical probe sterilizer including a main body having a sterilizing chamber configured to accommodate a medical probe to be sterilized therein and configured to be opened and closed, a probe holder installed on the main body and configured to hold the medical probe to be located inside the sterilizing chamber, a UV sterilization unit configured to sterilize the medical probe in the sterilizing chamber by using UV light, a plasma sterilization processing unit configured to sterilize the medical probe in the sterilizing chamber by using plasma, and a sealer configured to prevent leakage of the plasma to the outside of the sterilizing chamber.

## Description

### Technical Field

The present disclosure relates generally to a medical probe sterilizer. More particularly, the present disclosure relates to a medical probe sterilizer capable of sterilizing and disinfecting a medical probe for ultrasound examination.

### Background Art

In general, ultrasound examination is a process for diagnosing the presence or absence of diseases in organs inside the human body using harmless ultrasound waves, and is used in various medical fields because the examination is relatively inexpensive, simple, and safe (non-radiation). In Korea, the number of ultrasound examinations has been rapidly increasing due to national health insurance coverage.

Since ultrasound probes come into contact with an unspecified number of patients during the examination, various pathogens exist on the probe when the probes do not undergo periodic sterilization after the examination. In addition, since the probe is held by the user's hand during the examination, it can become a breeding ground for pathogens when proper hand hygiene is neglected by the user.

In particular, in the case of a vaginal ultrasound probe used in obstetrics and gynecology, it is inserted into the patient's vaginal cavity and comes into direct contact with the vaginal mucosa and cervix. Therefore, contamination of the probe can cause not only various sexually transmitted infections but also human papillomavirus (HPV) infection, which is the cause of cervical cancer. For this reason, the vaginal ultrasound probe is considered a semi-critical medical device and requires high-level sterilization before the examination.

In obstetrics and gynecology, ultrasound examination is performed in the following manner. The probe is primarily sterilized before the examination, and then a disposable protective cover (condom) is put on the sterilized probe. This process has to be repeated for each patient before the examination.

Despite the guidelines for managing ultrasound probe sterilization from the Korea Disease Control and Prevention Agency to prevent the high risk of infection during vaginal ultrasound examination in obstetrics and gynecology, there are often cases where the sterilization process is omitted due to the long sterilization time of 20 minutes or more per cycle and the examination is carried out by replacing only the disposable protective cover.

As described above, in a situation where the medical probe is not sterilized primarily and only the disposable protective cover is repeatedly used, the medical probe may be contaminated by pathogens when the user does not properly manage his or her hands, thereby making it meaningless to put the protective cover on the probe.

In particular, since the user repeatedly touches a handle of the probe, when the contaminated medical probe is repeatedly used without sterilization and disinfection processing, there is a possibility that the probe becomes a breeding ground for pathogens that infect the next patient. Also, there is another possibility that users who manipulate the medical probe are exposed to infection.

In view of this, an example of a device capable of sterilizing an ultrasound probe, i.e., a medical probe, is disclosed in Korean Utility Model Application Publication No. 20-2014-0003473.

According to the related-art technology, a probe is held inside a casing and sterilized using a UV lamp.

However, the related-art technology configured as described above has a limitation in sterilizing the probe with UV light, and in particular, in effectively sterilizing and disinfecting pathogens and viruses. That is, since effective sterilization cannot be guaranteed in an area where UV light does not reach, there is a limit to uniformly sterilizing gaps of the probe that are not exposed.

In addition, since UV light can only physically sterilize the probe, there is a limit to completely sterilizing the probe.

### Disclosure

### Technical Problem

Accordingly, the present disclosure has been made keeping in mind the above problems occurring in the related art, and an objective of the present disclosure is to provide a medical probe sterilizer capable of physically and chemically sterilizing and disinfecting a medical probe by means of UV light and plasma.

### Technical Solution

In order to accomplish the above objective, according to one aspect of the present disclosure, there is provided a medical probe sterilizer including: a main body including a sterilizing chamber configured to accommodate a medical probe to be sterilized therein and configured to be opened and closed; a probe holder installed on the main body and configured to hold the medical probe to be located inside the sterilizing chamber; a UV sterilization unit configured to sterilize the medical probe in the sterilizing chamber by using UV light; a plasma sterilization processing unit configured to sterilize the medical probe in the sterilizing chamber by using plasma; and a sealer configured to prevent leakage of the plasma to an outside of the sterilizing chamber.

With this configuration, the medical probe may be physically and chemically sterilized and disinfected with UV light and plasma.

Here, the main body may include: a housing having the plasma sterilization processing unit installed therein and having the probe holder and the UV sterilization unit installed on an outer front surface thereof; and a chamber door installed on the outer front surface of the housing, configured to be opened and closed to cover the medical probe held by the probe holder and the UV sterilization unit, and configured to form the sterilizing chamber between the housing in a closed state.

With this configuration, the medical probe to be sterilized may be easily attached and detached by easily opening and closing the sterilizing chamber.

In addition, the probe holder may include: a first holding member fixedly installed to protrude from the front surface of the housing to be located inside the sterilizing chamber and configured to hold a cable of the medical probe; and a second holding member located above the first holding member, fixed outside the sterilizing chamber, and configured to support the cable of the medical probe.

With this configuration, the medical probe to be sterilized may be held and fixed in a stable position in the sterilizing chamber.

In addition, the probe holder may further include a cable clamp coupled with the cable of the medical probe and configured to be held on the second holding member to support a load of the medical probe.

With this configuration, even when the size of the cable is different for each type of medical probe, the probe may be sterilized in a stably held state.

In addition, the UV sterilization unit may include: a front sterilization part disposed on the front surface of the housing and configured to emit the UV light; a lower sterilization part disposed in a lower part of an inside of the sterilizing chamber, protruding from the front surface of the housing, and configured to emit the UV light to a lower portion of the medical probe; and a pair of side sterilization parts disposed on opposite sides of the front sterilization part, protruding from the front surface of the housing, and configured to emit the UV light to side portions of the medical probe.

With this configuration, the medical probe held in the sterilizing chamber may be effectively sterilized by emitting UV light in various directions.

In addition, the sealer may include: a cable sealing holder installed to protrude from the front surface of the housing and having a cable mounting slit configured to allow the cable of the medical probe to be inserted and passed therethrough; and a sealing member installed on a tightly engaging edge of the chamber door configured to come into contact with the housing and configured to come into tight contact with the front surface of the housing and the cable sealing holder when the chamber door is closed.

With this configuration, the medical probe with the cable connected may be placed inside the sterilizing chamber and safely sterilized with UV light and plasma in a state of being completely blocked from the outside.

In addition, the plasma sterilization processing unit may include: a plasma generator installed inside the housing and configured to generate and supply the plasma to the sterilizing chamber; a plasma recovery pump installed inside the housing and configured to suction and recover the plasma supplied to the sterilizing chamber; and a filter installed on a recovery path of the plasma recovered by the plasma recovery pump and configured to filter out and remove ozone.

With this configuration, when the plasma used for sterilization is recovered, ozone may be filtered out safely.

In addition, the cable sealing holder may be made of a material that is elastically deformable by an external force.

With this configuration, a sealed state of the probe may be maintained more reliably.

### Advantageous Effects

According to a medical probe sterilizer according to the present disclosure, a medical probe can be physically and chemically sterilized and disinfected by means of UV light and plasma. Thus, the entire surface of the medical probe can be uniformly sterilized and disinfected.

In addition, by providing sealer that can completely block the sterilizing chamber from the outside in a state where the medical probe with a cable connected is held inside the sterilizing chamber, the probe can be stably sterilized while blocking leakage of plasma to the outside of the sterilizing chamber.

In addition, even when the size of the cable is different for each type of medical probe, the probe can be stably held and fixed in the sterilizing chamber.

As described above, through automatic sterilization and disinfection of the medical probe, it is possible to enable easy maintenance and reduce labor costs.

### Description of Drawings

FIG. 1 is a perspective view illustrating a medical probe sterilizer according to an embodiment of the present disclosure.
FIGS. 2 and 3 are exploded perspective views illustrating the medical probe sterilizer illustrated in FIG. 1.
FIG. 4 is a front view illustrating the medical probe sterilizer illustrated in FIG. 1.
FIG. 5 is a sectional view taken along line I - I of FIG. 4.
FIG. 6 is a plan view illustrating the medical probe sterilizer illustrated in FIG. 1.
FIG. 7 is a sectional view taken along line II-II of FIG. 6.
FIG. 8 is a perspective view illustrating a holding member illustrated in FIG. 2.
FIG. 9 is a partially sectional assembled view illustrating a sealer illustrated in FIG. 1.

### Best Mode

Hereinafter, a medical probe sterilizer according to an embodiment of the present disclosure will be described in detail with reference to the accompanying drawings.

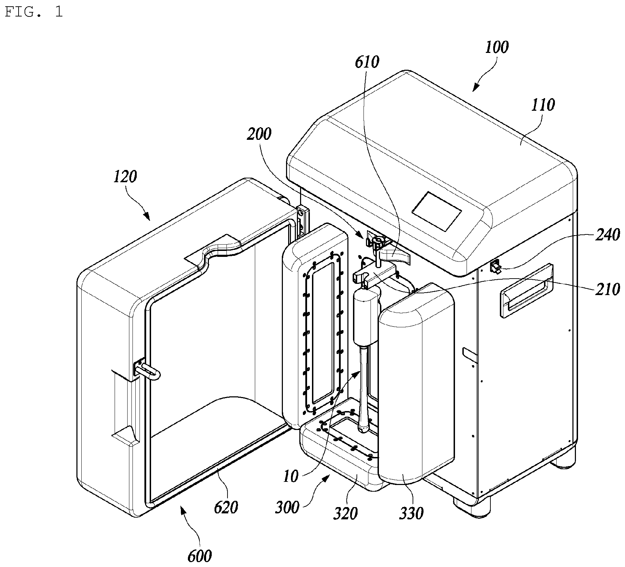

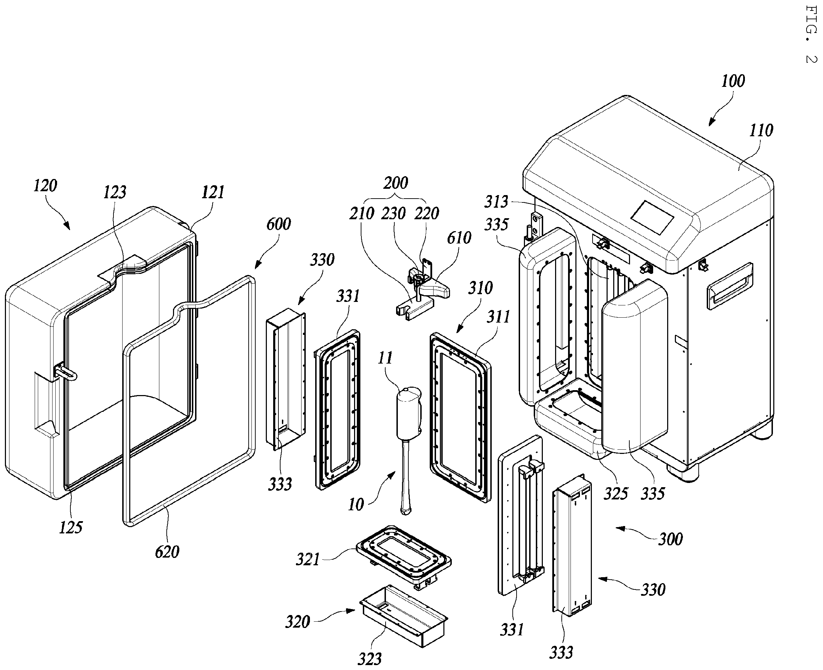

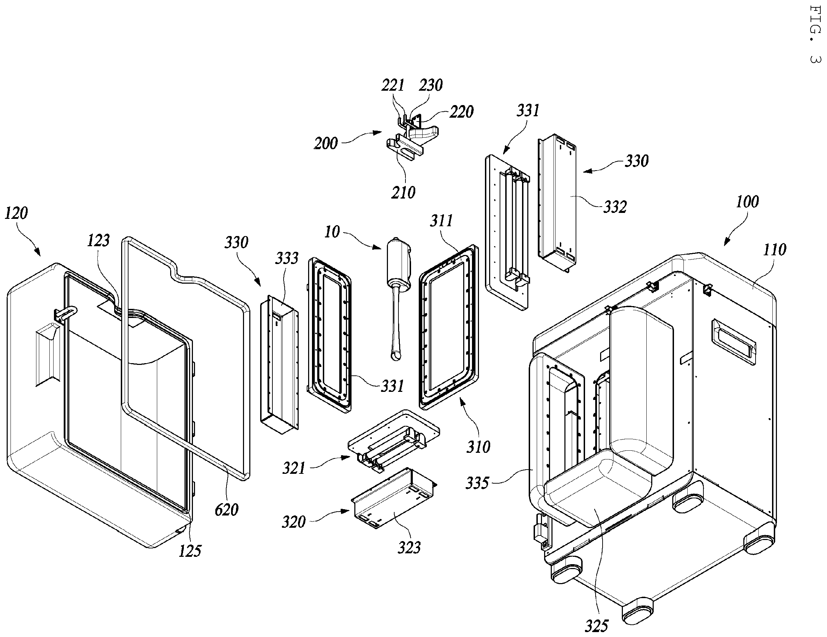

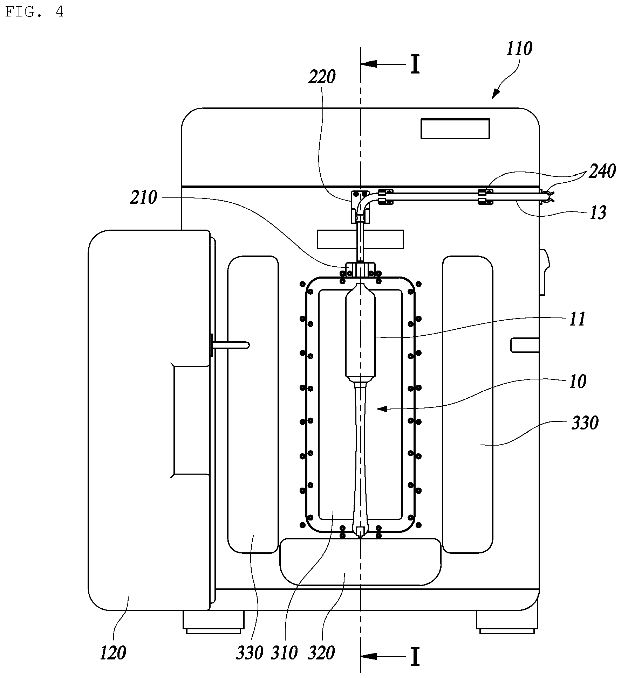

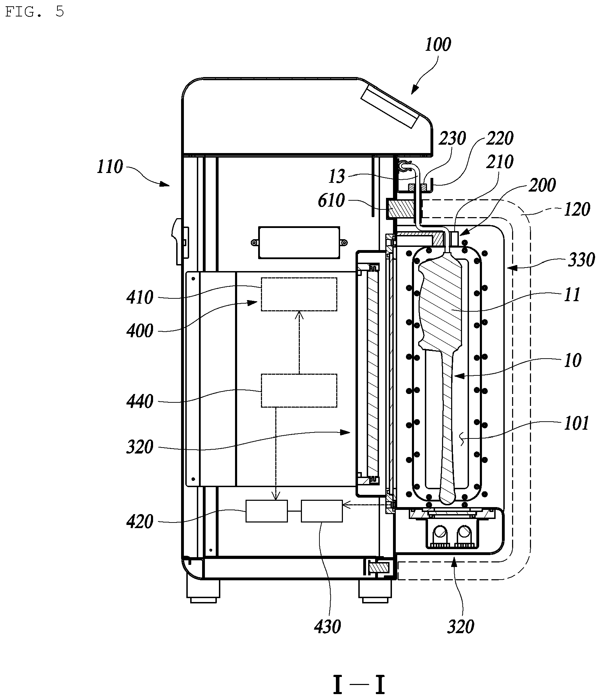

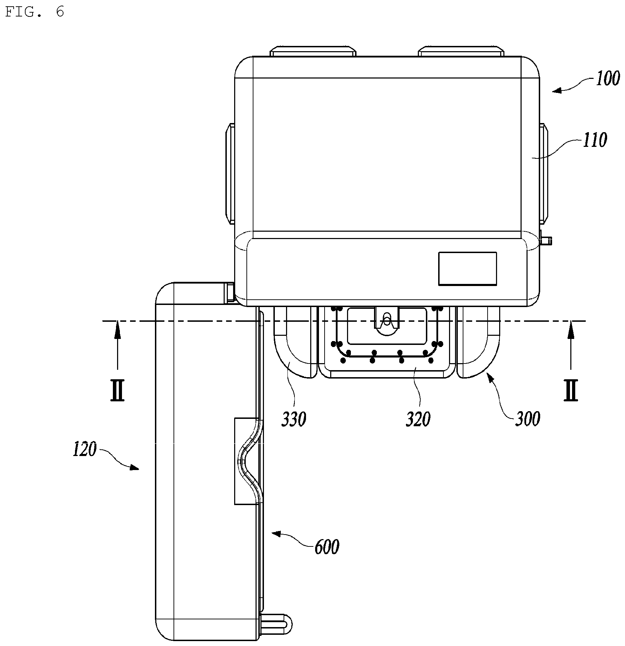

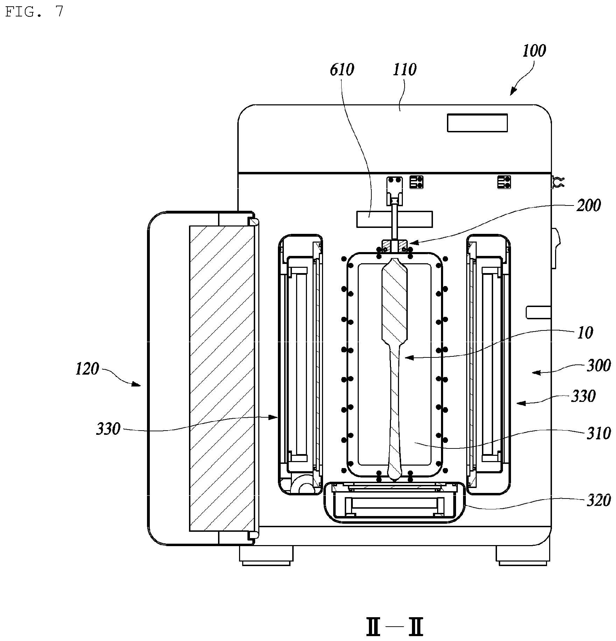

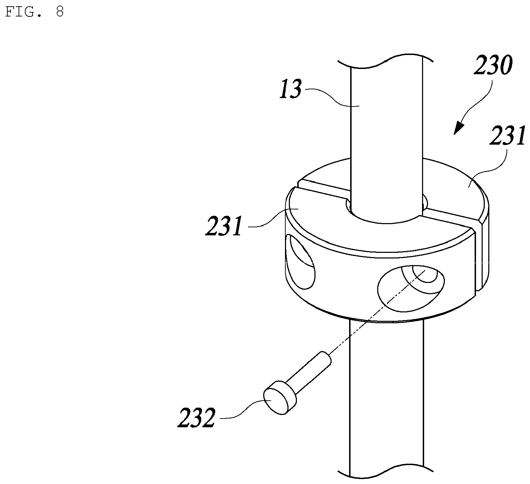

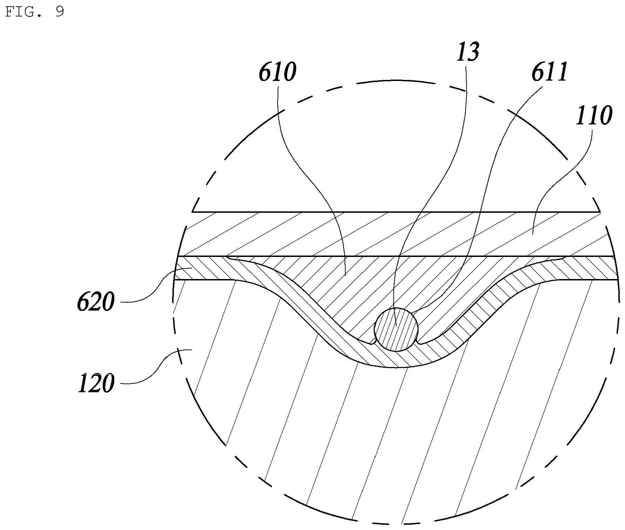

Referring to FIGS. 1 to 9, the medical probe sterilizer according to the embodiment of the present disclosure includes: a main body 100 having a sterilizing chamber 101 configured to accommodate a medical probe 10 therein; a probe holder 200 installed inside the sterilizing chamber 101 to support the medical probe 10; a UV sterilization unit 300 for physically sterilizing the medical probe 10 in the sterilizing chamber 101; a plasma sterilization processing unit 400 for sterilizing the medical probe 10 in the sterilizing chamber 101 by using plasma; and a sealer 600 for preventing leakage of plasma to the outside of the sterilizing chamber 101.

The main body 100 includes a housing 110, and a chamber door 120 coupled to a front surface of the housing 110 to be opened and closed to form the sterilizing chamber 101.

The housing 110 has a casing structure having a predetermined space therein, has the plasma sterilization processing unit 400 and a controller 440 installed therein. The chamber door 120 is coupled to the front surface of the housing 110 to be opened and closed. The probe holder 200 and the UV sterilization unit 300 are installed on a front surface of the main body 100 covered by the chamber door 120.

The probe holder 200 includes: a first holding member 210 fixedly installed to protrude from an upper portion of the front surface of the housing 110; a second holding member 220 located above the first holding member 210 and fixed outside the sterilizing chamber 101; and a cable clamp 230 coupled with a cable 13 of the probe 10 and held on the second holding member 220.

The first holding member 210 is installed to be located in an upper part of an inside of the sterilizing chamber 101, and has a front end provided with a cable coupling recess 211 in which the cable adjacent to a handle 11 of the probe 10 is forcibly inserted and supported.

The second holding member 220 is installed at a higher position than the first holding member 210, and is located outside the sterilizing chamber 101 so as to be exposed to the outside in a state in which the chamber door 120 is closed. The second holding member 220 is fixedly installed on the front surface of the main body 100 by a fastening member such as a bolt, and has a pair of support ribs 221 protruding forward. The pair of support ribs 221 are spaced apart from each other side by side, and have respective front ends extending and bent upward. The cable 13 of the probe 10 is passed between the support ribs 221, and the cable clamp 230 coupled with the cable 13 is held and supported on the support ribs 221. The cable clamp 230 held on the support ribs 221 is stably fixed without being separated by the bent front ends of the support ribs 221, so that the probe 10 is sterilized in a stably held state.

The cable clamp 230 fixes the cable 13 of the probe 10 in a clamping manner so that the cable 13 is stably fixed to the second holding member 220. That is, the size of the cable may be different for each type of probe 10. In view of this, the separate cable clamp 230 is coupled with the cable 13, and the cable clamp 230 is held and fixed to the second holding member 220. Thus, even when the cable 13 is not firmly fixed to the first holding member 210, the probe 10 is accommodated in a stable position in the space within the sterilizing chamber 101 and sterilized. The cable clamp 230 has a ring shape, and may include a pair of clamping members 231 each of which having at least one cut end and coupled in a state of clamping the cable 13. The pair of clamping members 231 may be opened to insert the cable 13 therebetween and coupled by a separate fastening member 232. Alternatively, the clamping members 231 may be coupled by an integral hook (not illustrated) in a one-touch manner. With this structure, the cable 13 is firmly fixed in a clamping manner.

In addition, a plurality of cable holders 240 are installed on an outer surface of the housing 110 to fix the cable 13.

The UV sterilization unit 300 includes a front sterilization part 310, a lower sterilization part 320, and a pair of side sterilization part 330.

The front surface sterilization part 310 includes a front optical module 311 installed on the front surface of the housing 110, and a front light reflector 313 is disposed on a rear surface of the front optical module 311 to reflect forward light generated from the front optical module 311. The front light reflector 313 is inserted and mounted in the front surface of the housing 110. The front light reflector 313 is coupled to the front light module 311.

The lower sterilization part 320 includes: a lower optical module 321 disposed in a lower part of the inside of the sterilizing chamber 101; a lower light reflector 323 disposed below the lower optical module 321; and a lower mounting portion 325 installed to protrude from a lower portion of the front surface of the housing 110 and to which the lower light reflector 323 is mounted.

The lower mounting portion 325 is installed to protrude from the front surface of the housing 110, and has an upper surface provided with a mounting recess in which the lower light reflector 323 is mounted.

A pair of side sterilization parts 330 are installed to face each other to sterilize opposite sides of the probe 10 held by the probe holder 200 by using UV light. Each of the side sterilization parts 330 includes: a side optical module 331 disposed on each side of the front sterilization part 310; a side light reflector 333 installed on a rear surface of the side optical module 331 to support the side optical module 331; and a side mounting portion 335 to which the side light reflector 333 is mounted. The side mounting portion 335 is installed to protrude from the front surface of the housing 110, and preferably extends vertically to a length corresponding to the length of the probe 10. A surface of the side mounting portion 335 facing the probe 10 is provided with a mounting recess in which the side light reflector 333 is mounted. The UV sterilization unit 300 having the above configuration emits UV light to the entire area of the probe 10 held by the probe holder 200, thereby effectively sterilizing the probe 10.

In addition, it is preferable that a reflector is installed on an inner wall of the chamber door 120 to reflect UV light generated from the UV sterilization unit 300 to focus on the probe 10.

The plasma sterilization processing unit 400 includes: a plasma generator 410 installed inside the housing 110 to generate and supply plasma to the sterilizing chamber 101; a plasma recovery pump 420 for suctioning and recovering the plasma supplied to the sterilizing chamber 101; a filter 430 installed on a recovery path of the plasma recovered by the plasma recovery pump 420 to filter out and remove ozone; and the controller 440.

The plasma generator 410 is driven and controlled by the controller 440 to generate plasma and supply the generated plasma into the sterilizing chamber 101 through a separate path. Preferably, the plasma is supplied from the upper part of the inside of the sterilizing chamber 101. To this end, one or a plurality of plasma discharge holes for supplying plasma are formed on the upper portion of the front surface of the housing 110.

The plasma recovery pump 420 is driven and controlled by the controller 440 to suction and recover plasma in the sterilizing chamber 101 through a plasma recovery line. Here, the plasma recovery pump 420 is operated to recover plasma after a set time has elapsed after driving the plasma generator 410. The filter 430 is installed on the plasma recovery line to filter out and remove ozone in the process of recovering plasma. A plasma recovery hole for recovering plasma recovered by the plasma recovery pump 420 is formed in the lower part of the sterilizing chamber 101. Here, it is preferable that one or a plurality of plasma recovery holes for recovering plasma are formed on a lower portion of the front surface of the housing 110.

The controller 440 drives and controls the UV sterilization unit 300 to sterilize the probe 10 with UV light when an operation signal is input with the chamber door 120 closed after the probe 10 to be sterilized is mounted in the sterilizing chamber 101. In addition, the controller 440 drives and controls the plasma generator 410 for a set time before or after sterilization by the UV sterilization unit 300 to supply plasma and sterilize the probe 10 with the plasma for a set time. After the set time has elapsed, the controller 440 drives and controls the plasma recovery pump 420 for a set time to safely recover and process plasma and ozone, thereby safely and effectively sterilize the probe 10.

The filter 430 is installed on the plasma recovery path and destroys ozone included in the recovered plasma to become a stable state. The filter 430 may include an activated carbon filter.

As described above, sterilization of the probe using plasma can completely chemically sterilize even an area that cannot be sterilized by UV light, thereby increasing reliability of sterilization processing of the probe 10 and preventing secondary spread of infection.

The sealer 600 includes a cable sealing holder 610 installed to protrude from the front surface of the housing 110, and a sealing member 620 installed on a tightly engaging edge of the chamber door 120. The cable sealing holder 610 protrudes from the front surface of the housing 110 so as to come into contact with the tightly engaging edge of the chamber door 120 when the chamber door 120 is closed to come into tight contact with the front surface of the housing 110. The cable sealing holder 610 has a curved surface coming into contact with the chamber door 120, and has a cable mounting slit 611 formed centrally and into which the cable 13 is press-fitted. The cable sealing holder 610 is preferably made of a material that is elastically deformable by an external force, for example, a material such as silicone or urethane. Thus, in a closed state of the chamber door 120, a sealed state is maintained more reliably by elastic deformation of the sealing member 620 and the cable sealing holder 610 with the cable 13 of the probe located between the sealing member 620 and the cable sealing holder 610.

The sealing member 620 is mounted in a sealing member mounting groove 125 formed on the tightly engaging edge of the chamber door 120 to provide sealing between the chamber door 120 and the housing 110. Preferably, the sealing member 620 includes a rubber packing. In addition, a concave portion 123 is formed in a curved shape on the tightly engaging edge of the chamber door 120 to correspond to a protruding shape of the cable sealing holder 610. The concave portion 123 may be formed to be concave on the tightly engaging edge of the chamber door 120, or may be provided by processing and combining a separate part.

According to the sealer 600 having the above configuration, the probe 10 is mounted in the sterilizing chamber 101, and the cable 13 is held on the cable sealing holder 610 by passing therethrough. Then, when the chamber door 120 is closed, the sealing member 620 comes into tight contact with the front surface of the housing 110 and the cable sealing holder 610, thereby completely blocking sealing the sterilizing chamber 101. In particular, since the cable sealing holder 610 has a curved outer surface, a contact force between the cable sealing holder 610 and the sealing member 620 is improved and generation of gaps therebetween is prevented. Also, the elastically deformed sealing member 620 is pushed into the entrance of the cable mounting slit 611, thereby providing a complete sealing without gaps.

As described above, according to the medical probe sterilizer according to the present disclosure, the probe 10 to be sterilized is held and fixed in a stable position in the sterilized chamber 101 through the holder 200. After holding the probe 10, the chamber door 120 is closed to block the door chamber 101 from the outside. Here, a locking ring is installed on the chamber door 120, and a locking device (lock) for locking the chamber door is provided in the device housing 110 to prevent the chamber door from being arbitrarily opened. The locking device may be a known automatic locking device, and may be set to be automatically unlocked when sterilizing processing is completed.

Meanwhile, when the chamber door 120 is closed as described above, while the cable 130 extends from the inside to the outside of the sterilizing chamber 101, the sterilizing chamber 101 is completely blocked from the outside by the sealer 600.

In this state, the controller 440 drives the UV sterilization unit 300 for a set time to sterilize the probe 10 with UV light. After the set time has elapsed, the plasma generator 410 is driven to supply plasma into the sterilizing chamber 101 and sterilize the probe 10 with the plasma for a set time.

After the set time has elapsed, the controller 400 drives the plasma recovery pump 420 to recover the plasma in the sterilizing chamber 101. During recovery of the plasma, ozone is filtered out by the filter 430 to prevent the ozone from leaking to the outside.

As described above, since the probe 10 is not only physically sterilized using UV light and but also chemically sterilized using plasma, the probe 10 can be effectively sterilized and safely used.

In particular, since the sterilization processing is automatically performed under control of the controller 440 in a state where the probe 10, which is frequently and repeatedly used, is mounted in the sterilizing chamber 101, it is possible to solve lack of manpower of managers or medical personnel, reduce maintenance costs, and enable safe management. Therefore, there is an advantage in solving problems caused by poor management and contamination of the probe 10.

Although a specific embodiment of the present disclosure has been described and illustrated as described above, it will be understood by those skilled in the art to which the present disclosure pertains that the present disclosure is not limited to the described embodiment but may be variously changed and modified without departing from the spirit and scope of the present disclosure. Accordingly, the changes and modifications should not be individually understood from the technical spirit and aspects of the present disclosure and the modified embodiments fall within the scope of the claims of the present disclosure.

## Claims

1. A medical probe sterilizer comprising:
a main body comprising a sterilizing chamber configured to accommodate a medical probe to be sterilized therein and configured to be opened and closed;
a probe holder installed on the main body and configured to hold the medical probe to be located inside the sterilizing chamber;
a UV sterilization unit configured to sterilize the medical probe in the sterilizing chamber by using UV light;
a plasma sterilization processing unit configured to sterilize the medical probe in the sterilizing chamber by using plasma; and
a sealer configured to prevent leakage of the plasma to an outside of the sterilizing chamber.

2. The medical probe sterilizer of claim 1, wherein the main body comprises:
a housing having the plasma sterilization processing unit installed therein and having the probe holder and the UV sterilization unit installed on an outer front surface thereof; and
a chamber door installed on the outer front surface of the housing, configured to be opened and closed to cover the medical probe held by the probe holder and the UV sterilization unit, and configured to form the sterilizing chamber between the housing in a closed state.

3. The medical probe sterilizer of claim 2, wherein the probe holder comprises:
a first holding member fixedly installed to protrude from the front surface of the housing to be located inside the sterilizing chamber and configured to hold a cable of the medical probe; and
a second holding member located above the first holding member, fixed outside the sterilizing chamber, and configured to support the cable of the medical probe.

4. The medical probe sterilizer of claim 3, wherein the probe holder further comprises a cable clamp coupled with the cable of the medical probe and configured to be held on the second holding member to support a load of the medical probe.

5. The medical probe sterilizer of claim 2, wherein the UV sterilization unit comprises:
a front sterilization part disposed on the front surface of the housing and configured to emit the UV light;
a lower sterilization part disposed in a lower part of an inside of the sterilizing chamber, protruding from the front surface of the housing, and configured to emit the UV light to a lower portion of the medical probe; and
a pair of side sterilization parts disposed on opposite sides of the front sterilization part, protruding from the front surface of the housing, and configured to emit the UV light to side portions of the medical probe.

6. The medical probe sterilizer of any one of claims 2 to 5, wherein the sealer comprises:
a cable sealing holder installed to protrude from the front surface of the housing and having a cable mounting slit configured to allow the cable of the medical probe to be inserted and passed therethrough; and
a sealing member installed on a tightly engaging edge of the chamber door configured to come into contact with the housing and configured to come into tight contact with the front surface of the housing and the cable sealing holder when the chamber door is closed.

7. The medical probe sterilizer of any one of claims 2 to 5, wherein the plasma sterilization processing unit comprises:
a plasma generator installed inside the housing and configured to generate and supply the plasma to the sterilizing chamber;
a plasma recovery pump installed inside the housing and configured to suction and recover the plasma supplied to the sterilizing chamber; and
a filter installed on a recovery path of the plasma recovered by the plasma recovery pump and configured to filter out and remove ozone.

8. The medical probe sterilizer of claim 6, wherein the cable sealing holder is made of a material that is elastically deformable by an external force.
